# FASCICULE DE BREVET EUROPEEN

(11) **EP 0 758 231 B1**
(45) Date de publication et mention de la délivrance du brevet: **11.07.2001**
(21) Numéro de dépôt: 95918046.4
(22) Date de dépôt: 24.04.1995
(51) Int. Cl.: A61K 31/335, A61K 47/24

(54) **COMPOSITIONS PHARMACEUTIQUES A BASE DE DERIVES DE LA CLASSE DES TAXANES**
TAXANDERIVATE ENTHALTENDE ARZNEIZUBEREITUNGEN
TAXANE CLASS DERIVATIVE BASED PHARMACEUTICAL COMPOSITIONS

(30) Priorité: 25.04.1994 FR 9404951
(43) Date de publication de la demande: 19.02.1997
(73) Titulaire: Aventis Pharma S.A., 92160 Antony (FR)
(72) Inventeur: DURR, Manfred, 50129 Bergheim-Glessen (DE); HAGER, Jörg-Christian, 50827 Köln (DE); WENDEL, Armin, 50859 Köln (DE)
(86) Numéro de dépôt international: FR9500532
(87) Numéro de publication internationale: WO9528923

(56) Documents cités:
- EP-A- 0 118 316
- WO-A-93/18751

## Description

La présente invention concerne une composition pharmaceutique à usage injectable, comprenant un agent thérapeutique antitumoral appartenant à la classe des taxoïdes.

Les principes actifs de la classe des taxoïdes sont des produits injectables, mais dont la solubilité dans l'eau est particulièrement faible et rend ainsi très difficile la constitution d'une préparation pour administration parentérale qui soit acceptable du point de vue thérapeutique.

La classe des taxoïdes comprend plus particulièrement le Taxotère® (docétaxel) ainsi que tous les dérivés de ce produit.

Parmi les dérivés du docétaxel peuvent être notamment cités les produits de formule générale : 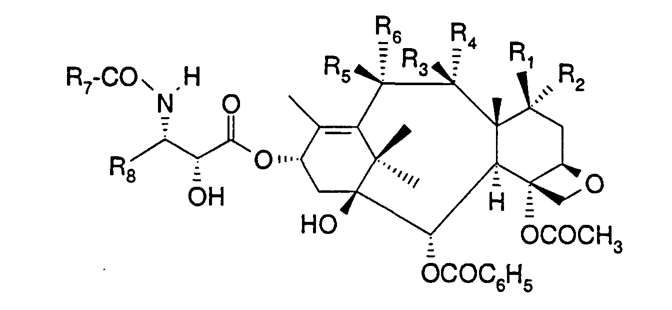 dans laquelle :
- les symboles R₁ et R₂ représentent chacun un atome d'hydrogène, ou bien un des radicaux R₁ ou R₂ représente un atome d'hydrogène, et l'autre représente un radical hydroxy, acyloxy ou acylcarbonyloxy, ou bien R₂ représente un atome d'hydrogène et R₁ forme avec l'atome de carbone du radical méthyle en α une liaison, de façon à former un cycle cyclopropane,
- l'un des symboles R₃ ou R₄ représente un atome d'hydrogène, et l'autre représente un radical hydroxy, ou bien R₃ et R₄ forment ensemble un radical oxo,
- les symboles R₅ et R₆ représentent chacun un atome d'hydrogène, ou bien un des symboles R₅ ou R₆ représente un atome d'hydrogène et l'autre représente un radical hydroxy, acyloxy, acylcarbonyloxy ou alcoxyméthylcarbonyloxy, ou bien R₅ et R₆ forment ensemble un radical oxo,
- le symbole R₇ représente un radical alcoxy, alcényloxy ou cycloalcoyloxy, et
- R₈ représente un radical alcoyle, alcényle droit ou ramifié, alcynyle droit ou ramifié, cycloalcoyle contenant 3 à 6 atomes de carbone, ou bien représente un radical phényle éventuellement substitué par un ou plusieurs atomes ou radicaux, identiques ou différents, choisis parmi les atomes; d'halogène et les radicaux alcoyles, alcoxy, dialcoylamino, acylamino, alcoxycarbonylamino ou trifluorométhyle, ou un radical hétérocyclique aromatique à 5 chaînons contenant un ou plusieurs hétéroatomes, identiques ou différents, choisis parmi les atomes d'azote, d'oxygène et de soufre,
étant entendu que les radicaux alcoyles et les portions alcoyles des autres radicaux contiennent 1 à 8 atomes de carbone en chaîne droite ou ramifiée et que les radicaux alcényles ou alcynyles contiennent 2 à 8 atomes de carbone.

Les taxoïdes qui peuvent être plus particulièrement utilisés dans le cadre de la présente invention sont les dérivés mentionnés ci-dessus pour lesquels R₂ représentant un atome d'hydrogène, R₁ représente un atome d'hydrogène ou un radical hydroxy, ou bien R₁ forme avec l'atome de carbone du radical méthyle en α une simple liaison, R₃ et R₄ forment ensemble un radical oxo, R₅ représente un atome d'hydrogène et R₆ représente un atome d'hydrogène ou un radical hydroxy, acétyloxy ou méthoxyacétyloxy ou bien R₅ et R₆ forment ensemble un radical oxo, R₇ représente un radical t-butoxy, et R₈ représente un radical isobutyle, isobutényle, butényle, cyclohexyle, phényle, furyle-2, furyle-3, thiényle-2, thiényle-3, thiazolyle-2, thiazolyle-4 ou thiazolyle-5.

Les dérivés de la classe des taxoïdes peuvent notamment être obtenus selon ou par analogie avec les méthodes décrites dans les demandes WO 92/09589, WO 93/06093, EP 534 708, EP 558 959, FR 2 697 019 ou selon ou par analogie avec la méthode décrite dans les exemples.

Jusqu'à présent différentes formulations avaient été mises au point comme notamment des compositions à base d'agent tensioactif et d'éthanol. L'éthanol, est le meilleur solvant pharmaceutique des agents de la classe des taxoïdes.

A titre d'exemple, selon la publication de Rowinsky, Lorraine, Cazenave et Donehower, Journal of the National Cancer Institute, 82(15), 1247-1259 (1990), on prépare une première solution, dite "solution mère", contenant environ 6 mg/ml de Taxol® dans un mélange solvant composé de:
- 50 % en volume d'éthanol
- 50 % en volume de Crémophor EL.

Lors de l'injection, cette solution est mélangée avec un liquide de perfusion contenant du chlorure de sodium ou du dextrose. Pour obtenir un mélange stable, d'un point de vue physique comme d'un point de vue chimique. Selon cet article il faut limiter la concentration en principe actif dans le soluté de perfusion à des concentrations n'excédant pas 0,6 mg/ml (voir page 1251 colonne 1, 3è paragraphe).

Il est néanmoins souhaitable de pouvoir injecter des doses suffisamment importantes de principe actif : généralement les cliniciens désirent pouvoir injecter des concentrations en principe actif comprises entre environ 0,3 et 1 mg/ml dans le liquide de perfusion. Malheureusement le facteur limitant est bien souvent lié à la teneur en excipients de la composition. En effet, au delà des doses mentionnées ci-dessus, des phénomènes de chocs anaphylactiques difficiles à maîtriser dûs pour l'essentiel au Crémophor peuvent apparaître (Rowinsky et al., J. Nat. Cancer Inst., 82 (15), 1250 1990), 2è colonne, dernier paragraphe ; Cancer Treat. Report., 71, 1171 - 1184 (1987)).

Selon la publication précitée, pour obtenir de telles concentrations (allant jusqu'à 1 mg/ml) il est nécessaire d'injecter des solutions contenant en même temps que le principe actif des concentrations en chacun des composés suivants, éthanol et surtout Crémophor, d'environ 8 g pour 100 ml de solution de perfusion. Le traitement demandant souvent l'administration de doses élevées de principe actif et la concentration du principe actif dans la solution étant relativement faible, l'injection de volumes importants a pour effet de provoquer des manifestations d'éthylisme durant le traitement, en plus des manifestations anaphylactiques.

Différentes recherches ont été effectuées dans le but de préparer des compositions pour administration parentérale de principes actifs insolubles dans l'eau, notamment à base de phospholipides (EP 118 316). Cependant, dans la mesure où il s'agissait de différents types de principes actifs, le problème à résoudre n'était pas de pouvoir accroître les concentrations en principe actif à des teneurs élevées dans les solutions destinées à l'injection. En conséquence, ces méthodes ne résolvaient pas le problème de la préparation d'une composition injectable améliorée, ayant un titre suffisant en principe actif pour un produit de la classe des taxoïdes.

Il a maintenant été trouvé que les agents anticancéreux appartenant à la classe des taxoïdes comme le docétaxel ou les dérivés du docétaxel, pouvaient être formulés à des taux exceptionnellement élevés, à l'état de composition pharmaceutique stable et ne présentant pas de problème d'intolérance. Les compositions pharmaceutiques selon l'invention comprennent un taxoïde dérivé du docétaxel, un ou plusieurs phospholipides insaturés et une faible quantité d'un ou plusieurs phospholipides négatifs. De préférence les compositions pharmaceutiques selon l'invention comprennent 3 à 15 mg/ml de taxoïde dérivé du docétaxel.

Les compositions selon l'invention peuvent être liquides, congelées ou lyophilisées. Les compositions liquides sont des solutions stables et limpides dans lesquelles aucune apparition de cristaux n'est observée. Les compositions congelées ou lyophilisées sont plus appropriées pour la conservation et permettent également de reconstituer des solutions stables et limpides, de forte concentration en principe actif de la classe des taxoïdes comme le docétaxel ou les dérivés du docétaxel.

Le terme "solutions stables" signifie des solutions stables à température ambiante dans lesquelles aucune apparition de particule de principe actif n'intervient pendant un temps supérieur à 8 semaines et pouvant aller jusqu'à 8 mois.

Les compositions lyophilisées sont un aspect préféré de l'invention. Elles présentent l'avantage d'une bonne stabilité physique et chimique et permettent surtout d'accroître la teneur en principe actif dans les compositions injectables, sans pour autant entraîner les problèmes d'intolérance précédemment observés. Il est ainsi possible, par application de la présente invention, d'accroître très fortement la solubilité de l'agent anticancéreux de la classe des taxoïdes comme le docétaxel ou les dérivés du docétaxel et de constituer des compositions injectables dans lesquelles la proportion relative du principe actif par rapport aux excipients est fortement augmentée.
De ce fait, il est maintenant possible de pallier les inconvénients liés à la présence d'excipients dont la toxicité n'est pas négligeable à des teneurs élevées.

Selon l'invention les phospholipides insaturés sont choisis parmi les phospholipides naturels, synthétiques ou semi-synthétiques; notamment les phospholipides naturels comme les phospholipides d'origine végétale (spécialement lécithines de colza, de tournesol ou de soja et par exemple des lécithines composées de différents phospholipides dans des proportions variées) ou animale (spécialement lécithine d'oeuf).

A titre d'exemple peuvent être notamment citées les phosphatidylcholines naturelles, comme notamment les phospholipons® : Phospholipon 80® , Phospholipon 90® , Phospholipon 100® . Peuvent être également cités les phosphatidyléthanolamines ; les phosphatidylinositols ; les phosphatidylsérines ; les phosphatidylglycérols ; l'acide phosphatidique ou des mélanges de ces phospholipides. Il est entendu que les phospholipides préférés sont les phospholipides de bonne qualité de pureté, c'est-à-dire ayant une pureté supérieure à 90 %.

Les phospholipides synthétiques insaturés peuvent être par exemple des phospholipides de structure : 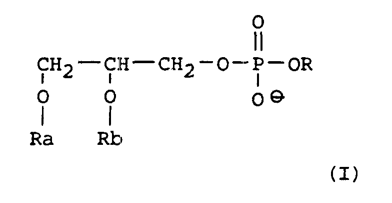 dans laquelle R est un radical alcoyle, substitué par amino ou par trialcoylammonio, (les radicaux alcoyle contenant 1 à 4 atomes de carbone) et Rₐ et R_{b} sont des atomes d'hydrogène ou des restes acyle saturés ou insaturés de chaînes grasses en C8 à C22 et sont identiques ou différents pourvu que l'un au moins soit un radical insaturé.

A titre d'exemple, plus particulièrement préférés sont les phospholipides insaturés pour lesquels R est aminoéthyle ou triméthylammonioéthyle, les phospholipides pour lesquels Rₐ et/ou R_{b} sont palmitoyle, stéaroyle, myristoyle, oléoyle, linoléoyle, linolénoyle, l'un au moins étant insaturé; et notamment les dérivés de phosphatidylcholine.

Les Phospholipons® sont des phospholipides naturels d'origine végétale extraits de la lécithine de soja qui contiennent un taux superieur à 70 % de chaînes acyle insaturées.

Selon l'invention les phospholipides négatifs sont choisis parmi les substances anioniques naturelles ou synthétiques comme par exemple les sels alcalins ou sels d'ammonium quaternaire de phosphatidylglycérol, de phosphatidylsérine, de phosphatidylinositol de l'acide phosphatidique ou leurs dérivés.

Les sels alcalins des phospholipides anioniques sont notamment les sels de sodium ou de potassium.

Les substances anioniques d'origine naturelle proviennent plus particulièrement du tournesol ou du soja.

Parmi les substances anioniques plus particulièrement préférés sont les sels du dimyristoylphosphatidylglycérol, du dipalmitoylphosphatidylglycérol ou du phosphatidylglycérol de soja ou leurs dérivés.

La préparation de la composition selon l'invention présente l'avantage considérable de ne pas faire intervenir de solvant organique toxique du point de vue pharmaceutique (comme par exemple les solvants chlorés) et dont l'élimination pourrait ne pas être totale dans la composition finale.

Selon l'invention, une dispersion homogène est formée par dissolution d'un ou plusieurs phospholipides insaturés, d'une faible quantité d'un ou plusieurs phospholipides négatifs et d'un principe actif de la classe des taxoïdes, dans un alcool (de préférence l'éthanol), suivie de l'évaporation de tout ou partie de l'alcool jusqu'à obtention d'un gel ou d'un liquide visqueux qui est repris par addition d'eau sous agitation puis homogénéisation. La dispersion homogène ainsi obtenue peut être congelée ou lyophilisée.

Il est entendu que l'homogénéisation peut être mise en oeuvre en plusieurs étapes répétées.

La dispersion homogène ainsi obtenue est stable et limpide. Elle présente l'avantage de contenir des particules de très faible dimension (diamètre moyen inférieur à 200 nm et de préférence inférieur à 100 nm), et peut de ce fait être soumise à une filtration stérilisante. Il est entendu que cette dispersion homogène entre dans le cadre de la présente invention.

Les lyophilisats obtenus peuvent être solubilisés extemporanément dans un milieu injectable au moment de l'emploi.

Lorsque la dispersion homogène préparée est soumise préalablement à une filtration stérilisante, la filtration s'effectue généralement sur filtre de 0,40 à 0,10 µm, de préférence de 0,30 µm à 0,20 µm et plus particulièrement sur filtre de 0,22 µm.

L'étape d'évaporation est mise en oeuvre de préférence sous atmosphère inerte, par exemple sous azote ou sous argon à une température inférieure à 45°C et de préférence à une température inférieure à 30°C. Il est avantageux d'opérer sous pression réduite. Il n'est pas toujours indispensable d'éliminer totalement l'alcool avant l'addition d'eau, un résidu d'alcool pouvant être éliminé par la suite après la formation de la dispersion.

La solution aqueuse peut éventuellement comprendre, en outre, des additifs. Le milieu peut être notamment additionné de composés non ioniques comme par exemple un agent cryoprotecteur destiné à empécher la reprécipitation du principe actif et/ou un agent destiné à ajuster l'isotonicité de la solution finale à injecter. Ces agents peuvent être choisis parmi des sucres (glucose, maltose, lactose, mannitol, sorbitol par exemple), des polymères [par exemple dextran (dextran 1500, dextran 40000), polyvinylpyrrolidones injectables, polyéthylèneglycol ...], des acides aminés (par exemple le glycocolle), ou tout autre agent pouvant exercer cette fonction. Elle peut également contenir un/(des) agent(s) conservateur(s). Les additifs peuvent être additionnés à différentes étapes de la préparation, cependant il est avantageux de les additionner dans la dispersion homogène.

La congélation peut être effectuée selon les techniques habituelles, et éventuellement de façon accélérée.

La lyophilisation est également effectuée selon les techniques habituelles.

La concentration en principe actif dans la composition pharmaceutique selon l'invention est comprise entre 3 et 15 mg/ml, sans qu'aucune apparition de particule ne se produise. De préférence la composition contient de 5 mg/ml à des valeurs supérieures à 10 mg/ml de taxoïde dérivé du docétaxel.

Le principe actif introduit dans la composition représente 1 à 30 % en poids par rapport à la somme des phospholipides introduits. De préférence le principe actif représente 3 à 20 % et plus spécialement 3,5 à 10 % en poids par rapport à la somme des phospholipides.

Le/les phospholipide(s) insaturé(s) sont de préférence dérivés de la phosphatidylcholine. Selon un aspect préféré de l'invention la phosphatidylcholine constitue de 70 à 100 % du phospholipide insaturé introduit.

Le phospholipide négatif est introduit en faible quantité. D'une manière générale, il est avantageux de l'introduire dans une proportion de 0,10 à 4 %, de préférence de 0,4 à 0,8 % et. plus particulièrement d'environ 0,5 % en poids par rapport à la totalité du/des phospholipide(s) insaturé(s).

Lorsque la composition obtenue est lyophilisée celle-ci peut être remise en solution au moment de l'emploi dans tout milieu injectable compatible et pharmaceutiquement acceptable. Le lyophilisat peut être avantageusement repris par de l'eau bidistillée de qualité injectable en volume équivalent au volume initial de la solution à lyophiliser. Lorsque la solution a été congelée (poche congelée par exemple), elle peut être décongelée au moment de l'emploi.

Les solutions ainsi obtenues présentent l'avantage d'être stables et de contenir une forte teneur en principe actif sans qu'aucune précipitation ou cristallisation ne se produise. Dans une autre alternative le lyophilisat peut être aussi remis en solution préalablement et la solution conservée jusqu'au moment de l'emploi. Le volume de milieu injectable additionné à ladite composition est de préférence identique au volume initial de la composition préalablement soumise à la lyophilisation. Lorsque la solution a été congelée elle peut être aussi stockée, après décongélation jusqu'à son utilisation.

Les exemples suivants, donnés à titre non limitatif illustrent des compositions selon l'invention.

### Exemple 1

1,0 g de docétaxel (Taxotère® ), 10,0 g de Phospholipon 90® et 0,05 g de sel de sodium de phosphatidyl glycérol sont mis en solution dans 90 ml d'éthanol puis agités jusqu'à dissolution complète. L'éthanol est évaporé sous atmosphère inerte (azote) et sous pression réduite 0,5 kPa à une température inférieure à 30°C, jusqu'à obtenir un solid pâteux exempt d'éthanol. Après addition d'eau jusqu'à un volume de 50,0 ml et dispersion du mélange par agitation, on obtient tout d'abord une dispersion d'aspect laiteux qui est homogénéisée jusqu'à obtention d'une dispersion fine et limpide et additionnée sous agitation de 50 ml d'une solution aqueuse contenant 30,0 g de maltose. La dispersion ainsi obtenue est soumise à une filtration stérilisante sur filtre de 0,22 µm.

La dispersion stérile est divisée en fractions de 10 ml dans des flacons de 20 ml puis lyophilisée.

Après reprise du lyophilisat par 10 ml d'eau bidistillée pour préparation injectable, on obtient immédiatement une solution stable et limpide (80 % de transparence mesurée sur photomètre à 660 nm).

### Exemple 2

On opère comme précédemment à l'exemple 1, mais à partir de 0,1 g de docétaxel, de 2,0 g de Phospholipon 90® et 0,01 g de sel de sodium de phosphatidyl glycérol. Après addition d'eau jusqu'à un volume de 16,7 ml, dispersion du mélange par agitation et homogénéisation, on obtient une dispersion limpide qui est additionnée de 3,3 ml d'une solution aqueuse contenant 2,0 g de maltose puis soumise à une filtration stérile.

La dispersion limpide est divisée en fractions de 4 ml dans des flacons de 10 ml puis lyophilisée.

On obtient un lyophilisat à partir duquel il est possible de reconstituer une solution parfaitement limpide et stable après addition de 4 ml d'eau contenant 0,9 % de chlorure de sodium.

Les mesures de stabilité montrent que la solution est toujours limpide après plus de 8 semaines à une température de 20°C.

Le diamètre de particules est d'environ 47 nm.

### Exemple 3

En opérant comme précédemment à l'exemple 1, mais à partir de 0,1 g de docétaxel, de 1,5 g de Phospholipon 90® et 0,075 g de sel de sodium de phosphatidyl glycérol. Après addition d'eau jusqu'à un volume de 12,5 ml, dispersion du mélange par agitation et homogénéisation, on obtient une dispersion limpide qui est additionnée de 2,5 ml d'une solution aqueuse contenant 1,5 g de maltose puis soumise à une filtration stérile.

La dispersion limpide est divisée en fractions de 1,5 ml dans des flacons de 5 ml puis lyophilisée.

On obtient un lyophilisat à partir duquel il est possible de reconstituer une solution parfaitement limpide et stable après addition de 1,5 ml d'eau contenant 0,9 % de chlorure de sodium.

Les mesures dé stabilité montrent que la solution est toujours limpide après plus de 8 semaines à une température de 20°C.

Le diamètre de particules est d'environ 71 nm.

### Exemple 4

En opérant comme précédemment à l'exemple 1, mais à partir de 0,1 g de docétaxel, de 2,0 g de Phospholipon 90® et 0,01 g de sel de sodium de phosphatidyl glycérol. L'éthanol est évaporé sous atmosphère inerte et sous pression réduite 0,15 kPa à une température inférieure à 30°C, jusqu'à obtenir un liquide visqueux. Après addition d'eau jusqu'à un volume de 20,0 ml, dispersion du mélange par agitation et évaporation de l'éthanol sous atmosphère inerte et sous pression réduite 0,4 kPa à une température inférieure à 30°C, on complète le volume à 20,0 ml par une nouvelle addition d'eau. On obtient ainsi une dispersion laiteuse qui est homogénéisée jusqu'à obtention d'une dispersion limpide. La dispersion est soumise à une filtration stérilisante puis répartie dans des ampoules de 2 ml.

La composition ainsi obtenue, exempte de particules, est congelée. Après décongélation, on obtient immédiatement une dispersion limpide.

### Exemple 5

En opérant comme précédemment à l'exemple 4, à partir de 0,1 g de docétaxel, de 1,5 g de Phospholipon 90® et 0,075 g de sel de sodium de phosphatidyl glycérol. Après addition d'eau jusqu'à un volume de 15,0 ml, dispersion du mélange par agitation et évaporation de l'éthanol, on complète le volume à 16,7 ml par une nouvelle addition d'eau. Après homogénéisation, on ajoute 3,3 ml d'une solution aqueuse contenant 2,0 g de maltose. La dispersion obtenue est soumise à une filtration stérilisante puis répartie en flacons de 2 ml et lyophilisée.

A partir du lyophilisat obtenu, il est possible de reconstituer une solution parfaitement limpide et stable après addition de 2,0 ml d'eau contenant 0,9 % de chlorure de sodium.

Les mesures de stabilité montrent que la solution est toujours limpide après 8 semaines à une température de 20°C.

### Exemple 6

0,87 g de t-butoxycarbonylamino-3' hydroxy-2' phényl-3' propionate-(2R,3S) de diacétoxy-4α,10β benzoyloxy-2α époxy-5β,20 hydroxy-1β méthylène-7β,8β oxo-9 nor-19 taxène-11 yl-13α, 17,4 g de Phospholipon 90® et 0,087 g de sel de sodium de phosphatidyl glycérol sont mis en solution dans 200 ml d'éthanol puis agités jusqu'à dissolution complète. L'éthanol est évaporé sous atmosphère inerte (azote) et sous pression réduite 0,5 kPa à une température inférieure à 30°C, jusqu'à obtenir un solide pâteux exempt d'éthanol. Après addition d'eau jusqu'à un volume de 143,0 ml et dispersion du mélange par agitation, on obtient tout d'abord une dispersion d'aspect laiteux qui est homogénéisée jusqu'à obtention d'une dispersion fine et limpide et additionnée sous agitation de 50 ml d'une solution aqueuse contenant 17,4 g de maltose. La dispersion ainsi obtenue est soumise à une filtration stérilisante sur filtre de 0,22 µm.

La dispersion stérile est divisée en fractions de 4,0 ml dans des flacons de 10 ml puis lyophilisée.

Après reprise du lyophilisat par 3,5 ml d'eau bidistillée pour préparation injectable, on obtient immédiatement une solution stable et limpide.

Cette solution est stable et limpide pendant plus de 8 semaines.

### Exemples 7 à 25

En opérant comme décrit dans les exemples ci-dessus on prépare des compositions analogues à partir des dérivés de la classe des taxoïdes mentionnés ci-après :
- t-butoxycarbonylamino-3' (fluoro-2 phényl)-3' hydroxy-2' propionate-(2R,3S) d'acétoxy-4 benzoyloxy-2α époxy-5β,20 trihydroxy-1β,7β,10β oxo-9 taxène-11 yl-13α;
- t-butoxycarbonylamino-3' (chloro-4 phényl)-3' hydroxy-2' propionate- (2R,3S) d'acétoxy-4 benzoyloxy-2α époxy-5β,20 trihydroxy-1β,7β,10β oxo-9 taxène-11 yl-13α;
- t-butoxycarbonylamino-3' (méthoxy-4 phényl)-3' hydroxy-2' propionate-(2R,3S) d'acétoxy-4 benzoyloxy-2α époxy-5β,20 trihydroxy-1β,7β,10β oxo-9 taxène-11 yl-13α;
- t-butoxycarbonylamino-3' (fluoro-4 phényl)-3' hydroxy-2' propionate-(2R,3S) d'acétoxy-4 benzoyloxy-2α époxy-5β,20 trihydroxy-1β,7β,10β oxo-9 taxène-11 yl-13α;
- adamantyloxycarbonylamino-3' hydroxy-2' phényl-3' propionate-(2R,3S) d'acétoxy-4 benzoyloxy-2α époxy-5β,20 trihydroxy-1β,7β,10β oxo-9 taxène-11 yl-13α;
- tert-pentyloxycarbonylamino-3' hydroxy-2' phényl-3' propionate-(2R,3S) d'acétoxy-4 benzoyloxy-2α époxy-5β,20 trihydroxy-1β,7β,10β oxo-9 taxène-11 yl-13α;
- (méthyl-1 cyclohexyl)oxycarbonylamino-3' hydroxy-2' phényl-3' propionate-(2R,3S) d'acétoxy-4 benzoyloxy-2α époxy-5β,20 trihydroxy-1β,7β,10β oxo-9 taxène-11 yl-13α;
- (méthyl-1 cyclopropyl)oxycarbonylamino-3' hydroxy-2' phényl-3' propionate-(2R,3S) d'acétoxy-4 benzoyloxy-2α époxy-5β,20 trihydroxy-1β,7β,10β oxo-9 taxène-11 yl-13α;
- (méthyl-1 cyclopentyl)oxycarbonylamino-3' hydroxy-2' phényl-3' propionate-(2R,3S) d'acétoxy-4 benzoyloxy-2α époxy-5β,20 trihydroxy-1β,7β,10β oxo-9 taxène-11 yl-13α;
- (diméthyl-1,1 propyne-2)yloxycarbonylamino-3' hydroxy-2' phényl-3' propionate-(2R,3S) d'acétoxy-4 benzoyloxy-2α époxy-5β,20 trihydroxy-1β,7β,10β oxo-9 taxène-11 yl-13α;
- t-butoxycarbonylamino-3' hydroxy-2' phényl-3' propionate-(2R,3S) d'acétoxy-4 benzoyloxy-2α époxy-5β,20 tétrahydroxy-1β,7β,9β,10β taxène-11 yl-13α;
- t-butoxycarbonylamino-3' hydroxy-2' phényl-3' propionate-(2R,3S) d'acétoxy-4 benzoyloxy-2α époxy-5β,20 dihydroxy-1β,7β oxo-9 taxène-11 yl-13α;
- t-butoxycarbonylamino-3' hydroxy-2' (thiényl-2)-3' propionate-(2R,3S) d'acétoxy-4 benzoyloxy-2α époxy-5β,20 trihydroxy-1β, 7β, 10β oxo-9 taxène-11 yl-13α;
- t-butoxycarbonylamino-3' (furyl-2)-3' hydroxy-2' propionate-(2R, 3S) d'acétoxy-4 benzoyloxy-2α époxy-5β,20 trihydroxy-1β,7β,10β oxo-9 taxène-11 yl-13α;
- t-butoxycarbonylamino-3' hydroxy-2' (thiényl-3)-3' propionate-(2R,3S) d'acétoxy-4 benzoyloxy-2α époxy-5β,20 trihydroxy-1β,7β,10β oxo-9 taxène-11 yl-13α;
- t-butoxycarbonylamino-3' hydroxy-2' phényl-3' propionate-(2R,3S) d'acétoxy-4 benzoyloxy-2α époxy-5β,20 dihydroxy-1β,10β oxo-9 taxène-11 yl-13α;
- t-butoxycarbonylamino-3' hydroxy-2' phényl-3' propionate-(2R,3S) d'acétoxy-4 benzoyloxy-2α époxy-5β,20 dihydroxy-1β,7β dioxo-9,10 taxène-11 yl-13α;
- t-butoxycarbonylamino-3' hydroxy-2' phényl-3' propionate-(2R,3S) d'acétoxy-4 benzoyloxy-2α époxy-5β,20 hydroxy-1β oxo-9 taxène-11 yl-13α;
- t-butoxycarbonylamino-3' hydroxy-2' phényl-3' propionate-(2R,3S) d'acétoxy-4 benzoyloxy-2α époxy-5β,20 dihydroxy-1β,10β méthylène-7β,8β oxo-9 nor-19 taxène-11 yl-13α.

### Exemple de préparation d'un dérivé de formule générale (I)

A une solution de 550 mg d'amino-3 hydroxy-2 phényl-3 propionate-(2R,3S) de diacétoxy-4α, 10β benzoyloxy-2α époxy-5β,20 hydroxy-1β méthylène-7β,8β oxo-9 nor-19 taxène-11 yle-13α dans 1 cm3 de dichlorométhane, maintenue sous atmosphère d'argon, on ajoute 76 mg d'hydrogénocarbonate de sodium puis goutte à goutte, à une température voisine de 20°C, une solution de 197 mg de dicarbonate de di-tert-butyle dans 1 cm3 de dichlorométhane. La solution obtenue est agitée pendant 15 heures à une température voisine de 20°C puis additionnée d'un mélange de 5 cm3 d'eau distillée et de 10 cm3 de dichlorométhane. La phase aqueuse est extraite par 5 cm3 de dichlorométhane. Les phases organiques rassemblées sont séchées sur sulfate de magnésium, filtrées puis concentrées à sec sous pression réduite (2,7 kPa) à 40°C. On obtient ainsi 780 mg d'une meringue blanche que l'on purifie par chromatographie à pression atmosphérique sur 50 g de silice (0,063-0,2 mm) contenus dans une colonne de 2,5 cm de diamètre en éluant avec un mélange méthanol-dichlorométhane (1-99 puis 2,5-97,5 en volumes) en recueillant des fractions de 10 cm3. Les fractions ne contenant que le produit cherché sont réunies et concentrées à sec sous pression réduite (2,7 kPa) à 40°C. On obtient ainsi 660 mg d'une meringue blanche. Un échantillon de 300 mg est purifié par chromatographie préparative sur 12 plaques de silice en couche mince (Kieselgel 60F254, Merck ; épaisseur 0,25 mm) en éluant avec un mélange méthanol-dichlorométhane (4-96 en volumes). Après élution de la zone correspondant au produit principal par un mélange méthanol-dichlorométhane (10-90 en volumes) puis évaporation des solvants sous pression réduite (0,27 k.Pa) à une température voisine de 40°C, on obtient 159,7 mg de tert-butoxycarbonylamino-3 hydroxy-2 phényl-3 propionate-(2R,3S) de diacétoxy-4α,10β benzoyloxy-2α époxy-5β,20 hydroxy-1β méthylène-7β, 8β oxo-9 nor-19 taxène-11 yle-13α sous forme d'une meringue blanche, dont les caractéristiques sont les suivantes :
- pouvoir rotatoire : [α]²⁰_{D} = -34° (c = 0,564 ; méthanol)
- spectre de R.M.N. du proton : (400 MHz ; CDCl₃; δ en ppm ; constantes de couplage J en Hz): 1,28 (s, 3H : -CH₃ 16 ou 17) ; 1,30 [s, 9H : -C(CH₃)₃]; 1,38 (mt, 1H : -H 7) ; 1,60 (s, 3H : -CH₃ 16 ou 17) ; 1,68 et 2,25 (t et m, 1H chacun : CH₂ du cyclopropane) ; 1,85 (s, 3H: -CH₃ 18); 2,10 et 2,45 (d et td, 1H chacun : -CH₂- en 6) ; 2,23 (s, 3H: -COCH₃ en 10) ; 2,22 et 2,40 (m, 1H chacun : -CH₂- en 14) ; 2,40 (s, 3H : -COCH₃ en 4) ; 3,28 (d, 1H : -OH en 2') ; 4,05 et 4,22 (d, 1H chacun : -CH₂- en 20) ; 4,10 (d, 1H : -H 3) ; 4,62 (s large, 1H: -H 2') ; 4,73 (d, 1H : -H 5) ; 5,29 (d large, 1H: -H 3'); 5,37 (d, 1H: -CONH-) ; 5,67 (d, 1H : -H en 2) ; 6,28 (t large, 1H : -H 13) ; 6,33 (s, 1H : -H 10) ; de 7,30 à 7,45 (mt, 5H : -C₆H₅ en 3') ; 7,51 [t, 2H : -OCOC₆H₅ (-H 3 et -H 5)] ; 7,61 [t, 1H : -OCOC₆H₅ (-H 4)]; 8,17 [d, 2H : -OCOC₆H₅ (-H 2 et -H 6)].

A partir de 1,6 g de tert-butoxycarbonyl-3 diméthyl-2,2 phényl-4 oxazolidinecarboxylate-5-(4S, 5R) de diacétoxy-4a, 10β benzoyloxy-2α époxy-5β,20 hydroxy-1β méthylène-7β, 8β oxo-9 nor-19 taxène-11 yle-13α , on obtient 1,14 g d'amino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α sous forme d'une meringue blanche.

A partir de 2,2 g de tert-butoxycarbonyl-3 diméthyl-2,2 phényl-4 oxazolidinecarboxylate-5-(4S,5R) de diacétoxy-4α, 10β benzoyloxy-2α époxy-5β,20 hydroxy-1β oxo-9 trifluorométhanesulfonate-7β taxène-11 yle-13α, on obtient 1,62 g de tert-butoxycarbonyl-3 diméthyl-2,2 phényl-4 oxazolidinecarboxylate-5-(4S,5R) de diacétoxy-4α, 10β benzoyloxy-2α époxy-5β,20 hydroxy-1β méthylène-7β,8β oxo-9 nor-19 taxène-11 yle-13 sous forme d'une meringue blanche.

A partir de 2,4 g de tert-butoxycarbonyl-3 diméthyl-2,2 phényl-4 oxazolidinecarboxylate-5-(4S,5R) de diacétoxy-4α,10β benzoyloxy-2α époxy-5β,20 dihydroxy-1β,7β oxo-9 taxène-11 yle-13α, on obtient 2,46 g de tert-butoxy-carbonyl-3 diméthyl-2,2 phényl-4 oxazolidine-carboxylate-5-(4S,5R) de diacétoxy-4α, 10β benzoyloxy-2α époxy-5β,20 hydroxy-1β oxo-9 trifluorométhanesulfonate-7β taxène-11 yle-13α sous forme d'une meringue blanche.

Le tert-butoxycarbonyl-3 diméthyl-2,2 phényl-4 oxazolidine-carboxylate-5-(4S, 5R) de diacétoxy-4α,10β benzoyloxy-2α époxy-5β,20 dihydroxy-1β,7β oxo-9 taxène-11 yle-13α est obtenu dans les conditions décrites dans la demande internationale WO 92/09589.

## Revendications

1. Composition pharmaceutique stable et fortement concentrée en principe actif de la classe des taxoïdes choisi parmi le docétaxel ou les dérivés du docétaxel, caractérisée en ce qu'elle comprend un agent thérapeutique de la classe des taxoïdes choisi parmi le docétaxel ou les dérivés du docétaxel, un ou plusieurs phospholipides insaturés et une faible quantité d'un ou plusieurs phospholipides négatifs.

2. Composition pharmaceutique selon la revendication 1, caractérisée en ce qu'elle comprend 3 à 15 mg/ml de l'agent thérapeutique de la classe des taxoïdes choisi parmi le docétaxel ou les dérivés du docétaxel.

3. Composition pharmaceutique selon l'une des revendications 1 ou 2, caractérisée en ce que l'agent thérapeutique de la classe des taxoïdes choisi parmi le docétaxel ou les dérivés du docétaxel est choisi parmi les produits de formule générale : 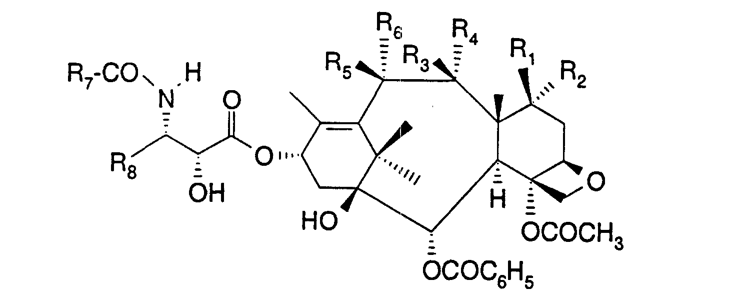 dans laquelle :
- les symboles R₁ et R₂ représentent chacun un atome d'hydrogène, ou bien un des radicaux R₁ ou R₂ représente un atome d'hydrogène, et l'autre représente un radical hydroxy, acyloxy ou acylcarbonyloxy, ou bien R₂ représente un atome d'hydrogène et R₁ forme avec l'atome de carbone du radical méthyle en α une liaison, de façon à former un cycle cyclopropane,
- l'un des symboles R₃ ou R₄ représente un atome d'hydrogène, et l'autre représente un radical hydroxy, ou bien R₃ et R₄ forment ensemble un radical oxo,
- les symboles R₅ et R₆ représentent chacun un atome d'hydrogène, ou bien un des symboles R₅ ou R₆ représente un atome d'hydrogène et l'autre représente un radical hydroxy, acyloxy, acylcarbonyloxy ou alcoxyméthylcarbonyloxy, ou bien R₅ et R₆ forment ensemble un radical oxo,
- le symbole R₇ représente un radical alcoxy, alcényloxy ou cycloalcoyloxy, et
- R₈ représente un radical alcoyle droit ou ramifié, alcényle droit ou ramifié, alcynyle droit ou ramifié, cycloalcoyle contenant 3 à 6 atomes de carbone, ou bien représente un radical phényle éventuellement substitué par un ou plusieurs atomes ou radicaux, identiques ou différents, choisis parmi les atomes d'halogène et les radicaux alcoyles, alcoxy, dialcoylamino, acylamino, alcoxycarbonylamino ou trifluorométhyle, ou un radical hétérocyclique aromatique à 5 chaînons contenant un ou plusieurs hétéroatomes, identiques ou différents, choisis parmi les atomes d'azote, d'oxygène et de soufre,
étant entendu que les radicaux alcoyles et les portions alcoyles des autres radicaux contiennent 1 à 8 atomes de carbone en chaîne droite ou ramifiée et que les radicaux alcényles ou alcynyles contiennent 2 à 8 atomes de carbone.

4. Composition pharmaceutique selon la revendication 1, 2 ou 3, caractérisée en ce que l'agent thérapeutique de la classe des taxoïdes choisi parmi le docétaxel ou les dérivés du docétaxel est choisi parmi les produits définis dans la revendication 2 pour lesquels R₂ représentant un atome d'hydrogène, R₁ représente un atome d'hydrogène ou un radical hydroxy, ou bien R₁ forme avec l'atome de carbone du radical méthyle en α une simple liaison, R₃ et R₄ forment ensemble un radical oxo, R₅ représente un atome d'hydrogène et R₆ représente un atome d'hydrogène ou un radical hydroxy, acétyloxy ou méthoxyacétyloxy ou bien R₅ et R₆ forment ensemble un radical oxo, R₇ représente un radical t-butoxy, et R₈ représente un radical isobutyle, isobutényle, butényle, cyclohexyle, phényle, furyle-2, furyle-3, thiényle-2, thiényle-3, thiazolyle-2, thiazolyle-4 ou thiazolyle-5.

5. Composition pharmaceutique selon l'une des revendications 1 à 4, caractérisée en ce que l'agent thérapeutique est le docétaxel.

6. Composition pharmaceutique selon l'une des revendications 1 à 4, caractérisée en ce que l'agent thérapeutique est le t-butoxycarbonyl-amino-3' hydroxy-2' phényl-3' propionate-(2R,3S) de diacétoxy-4α, 10β benzoyloxy-2α époxy-5β,20 hydroxy-1β méthylène-7β,8β oxo-9 nor-19 taxène-11 yl-13α.

7. Composition pharmaceutique selon l'une des revendications 1 à 6, caractérisée en ce que la concentration en principe actif est comprise entre 5 et des valeurs supérieures à 10 mg/ml.

8. Composition pharmaceutique selon l'une des revendications 1 à 7, caractérisée en ce que le phospholipide insaturé est un phospholipide naturel, synthétique ou semi-synthétique.

9. Composition pharmaceutique selon la revendication 8, caractérisée en ce que le phospholipide insaturé est un phospholipide naturel.

10. Composition pharmaceutique selon la revendication 8 ou 9, caractérisée en ce que le phospholipide naturel est un phospholipide d'origine végétale, en particulier de tournesol ou de soja.

11. Composition pharmaceutique selon l'une des revendications 1 à 10, caractérisée en ce que le phospholipide insaturé est constitué par 70 à 100 % de phosphatidylcholine.

12. Composition pharmaceutique selon la revendication 10 ou 11, caractérisée en ce que le phospholipide naturel d'origine végétale contient un taux superieur à 70 % de chaînes acyle insaturées.

13. Composition pharmaceutique selon l'une des revendications précédentes, caractérisée en ce que l'agent thérapeutique de la classe des taxoïdes choisi parmi le docétaxel ou les dérivés du docétaxel est introduit à raison de 1 à 30 % en poids par rapport à la somme des phospholipides.

14. Composition pharmaceutique selon la revendication 13, caractérisée en ce que l'agent thérapeutique est introduit à raison de 3 à 20 % en poids par rapport à la somme des phospholipides.

15. Composition pharmaceutique selon la revendication 13 ou 14, caractérisée en ce que l'agent thérapeutique est introduit à raison de 3,5 à 10 % en poids par rapport à la somme des phospholipides.

16. Composition pharmaceutique selon l'une des revendications précédentes, caractérisée en ce que le/les phospholipides négatifs sont choisis parmi les sels alcalins ou sels d'ammonium quaternaire de phosphatidylglycérol, de phosphatidylsérine, de phosphatidylinositol de l'acide phosphatidique ou leurs dérivés.

17. Composition pharmaceutique selon la revendication 16, caractérisée en ce que le/les phospholipides négatifs sont introduit à raison d'environ 0,1 à 4 % en poids par rapport à la totalité du/des phospholipides insaturés.

18. Composition pharmaceutique selon la revendication 16 ou 17, caractérisée en ce que le/les phospholipides négatifs sont introduit à raison d'environ 0,4 à 0,8 % en poids par rapport à la totalité du/des phospholipides insaturés.

19. Composition pharmaceutique selon l'une des revendications 16 à 18, caractérisée en ce que le/les phospholipides négatifs sont introduit à raison d'environ 0,5 % en poids par rapport à la totalité du/des phospholipides insaturés.

20. Composition pharmaceutique selon l'une des revendications précédentes, caractérisée en ce qu'il s'agit d'une dispersion homogène.

21. Composition pharmaceutique selon l'une des revendications précédentes, caractérisée en ce qu'elle peut être liquide, congelée ou lyophilisée.

22. Composition pharmaceutique selon l'une des revendications précédentes, caractérisée en ce qu'elle contient un agent cryoprotecteur et/ou un agent destiné à ajuster l'isotonicité de la solution finale à injecter.

23. Composition pharmaceutique selon la revendication 22, caractérisée en ce que l'agent est choisi parmi des sucres, des polymères ou des acides aminés.

24. Composition selon l'une des revendications précédentes, caractérisée en ce qu'elle contient des particules dont le diamètre moyen est inférieur à 200 nm.

25. Composition selon la revendication 24, caractérisée en ce qu'elle contient des particules dont le diamètre moyen est inférieur à 100 nm.

26. Un procédé de préparation d'une composition pharmaceutique selon l'une des revendications précédentes, caractérisé en ce que l'on forme une dispersion homogène par dissolution dans un alcool, d'un ou plusieurs phospholipides insaturés, d'une faible quantité d'un ou plusieurs phospholipides négatifs et d'un principe actif de la classe des taxoïdes choisi parmi le docétaxel ou les dérivés du docétaxel, évaporation de tout ou partie de l'alcool jusqu'à obtention d'un gel ou d'un liquide visqueux qui est repris par addition d'eau sous agitation puis homogénéisation, puis éventuellement éventuellement congèle ou lyophilise la dispersion obtenue.

27. Un procédé selon la revendication 26, caractérisé en ce que l'alcool est l'éthanol.

28. Procédé de préparation d'une dispersion homogène selon la revendication 20, caractérisé en ce que l'on soumet à une homogénéisation la dispersion des phospholipides et du principe actif de la classe des taxoïdes choisi parmi le docétaxel ou les dérivés du docétaxel, obtenue après addition d'eau.

29. Procédé selon la revendication 28 caractérisé en ce que l'homogénéisation est mise en oeuvre en plusieurs étapes répétées.

30. Un procédé selon l'une des revendications 26 à 29, caractérisé en ce que la dispersion homogène obtenue est soumise à une filtration stérilisante.

31. Utilisation d'une solution congelée ou lyophilisée selon la revendication 21, pour la préparation de solutions injectables stabilisées, prêtes à l'emploi.

## Patentansprüche

1. Stabile und an Wirkstoff der Taxoid-Klasse, der unter Docetaxel oder den Docetaxel-Derivaten ausgewählt ist, stark konzentrierte pharmazeutische Zusammensetzung, dadurch gekennzeichnet, dass sie ein Therapeutikum der Taxoid-Klasse, das unter Docetaxel oder den Docetaxel-Derivaten ausgewählt ist, ein oder mehrere ungesättigte Phospholipide und eine geringe Menge eines oder mehrerer negativer Phospholipide umfaßt.

2. Pharmazeutische Zusammensetzung gemäß Anspruch 1, dadurch gekennzeichnet, dass sie 3 bis 15 mg/ml Therapeutikum der Taxoid-Klasse, das unter Docetaxel oder den Docetaxel-Derivaten ausgewählt ist, umfaßt.

3. Pharmazeutische Zusammensetzung gemäß einem der Ansprüche 1 oder 2, dadurch gekennzeichnet, dass das Therapeutikum der Taxoid-Klasse, das unter Docetaxel oder den Docetaxel-Derivaten ausgewählt ist, unter den Produkten der allgemeinen Formel: 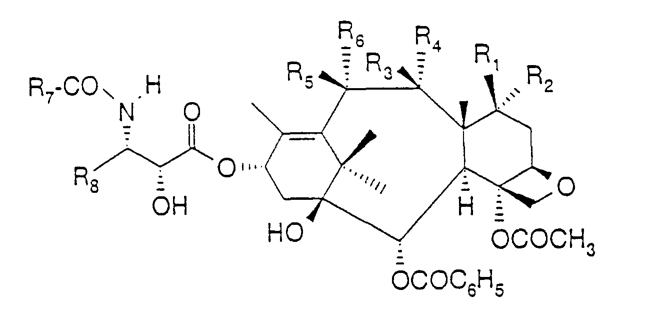 ausgewählt ist, in der:
- die Symbole R₁ und R₂ jeweils ein Wasserstoffatom darstellen oder auch einer der Reste R₁ oder R₂ ein Wasserstoffatom darstellt und der andere einen Hydroxy-, Acyloxy- oder Acylcarbonyloxyrest darstellt, oder auch R₂ ein Wasserstoffatom darstellt und R₁ mit dem Kohlenstoffatom des Methylrests in α-Stellung eine Bindung bildet, um einen Cyclopropanring zu bilden,
- das eine der Symbole R₃ oder R₄ ein Wasserstoffatom darstellt und das andere einen Hydroxyrest darstellt, oder auch R₃ und R₄ zusammen einen Oxorest bilden,
- die Symbole R₅ und R₆ jeweils ein Wasserstoffatom darstellen, oder auch eines der Symbole R₅ oder R₆ ein Wasserstoffatom darstellt und das andere einen Hydroxy-, Acyloxy- oder Acylcarbonyloxy- oder Alkoxymethylcarbonyloxyrest darstellt, oder auch R₅ und R₆ zusammen einen Oxorest bilden,
- das Symbol R₇ einen Alkoxy-, Alkenyloxy- oder Cycloalkyloxyrest darstellt, und
- R₈ einen geraden oder verzweigten Alkyl-, geraden oder verzweigten Alkenyl-, geraden oder verzweigten Alkinyl-, Cycloalkylrest mit 3 bis 6 Kohlenstoffatomen darstellt, oder auch einen Phenylrest, der gegebenenfalls mit einem oder mehreren identischen oder verschiedenen Atomen oder Resten, die unter den Halogenatomen und den Alkyl-, Alkoxy-, Dialkylamino-, Acylamino-, Alkoxycarbonylamino- oder Trifluormethylresten ausgewählt sind, substituiert ist, oder einen aromatischen heterocyclischen Rest mit 5 Kettengliedern, der ein oder mehrere identische oder verschiedene Heteroatome, die unter den Stickstoff-, Sauerstoff- und Schwefelatomen ausgewählt sind, enthält, darstellt,
wobei es sich versteht, dass die Alkylreste und die Alkylteile der anderen Reste 1 bis 8 Kohlenstoffatome in gerader oder verzweigter Kette enthalten und dass die Alkenyl- oder Alkinylreste 2 bis 8 Kohlenstoffatome enthalten.

4. Pharmazeutische Zusammensetzung gemäß Anspruch 1, 2 oder 3, dadurch gekennzeichnet, dass das Therapeutikum der Taxoid-Klasse, das unter Docetaxel oder den Docetaxel-Derivaten ausgewählt ist, unter den in Anspruch 2 definierten Produkten, für die R₂ ein Wasserstoffatom darstellt, R₁ ein Wasserstoffatom oder einen Hydroxyrest darstellt, oder auch R₁ mit dem Kohlenstoffatom des Methylrests in α-Stellung eine Einfachbindung bildet, R₃ und R₄ zusammen einen Oxorest bilden, R₅ ein Wasserstoffatom darstellt und R₆ ein Wasserstoffatom oder einen Hydroxy-, Acetyloxy- oder Methoxyacetyloxyrest darstellt, oder auch R₅ und R₆ zusammen einen Oxorest bilden, R₇ einen t-Butoxyrest darstellt und R₈ einen Isobutyl-, Isobutenyl-, Butenyl-, Cyclohexyl-, Phenyl-, 2-Furyl-, 3-Furyl-, 2-Thienyl-, 3-Thienyl-, 2-Thiazolyl-, 4-Thiazolyl- oder 5-Thiazolyl-Rest darstellt, ausgewählt ist.

5. Pharmazeutische Zusammensetzung gemäß einem der Ansprüche 1 bis 4, dadurch gekennzeichnet, dass das Therapeutikum Docetaxel ist.

6. Pharmazeutische Zusammensetzung gemäß einem der Ansprüche 1 bis 4, dadurch gekennzeichnet, dass das Therapeutikum (2R,3S)-3'-t-Butoxy-carbonylamino-2'-hydroxy-3'-phenyl-propionsäureester von 4α, 10β-Diacetoxy-2α-benzoyl-5β,20-epoxy-1β-hydroxy-7β,8β-methylen-9-oxo-19-nor-11-taxen-13-yl ist.

7. Pharmazeutische Zusammensetzung gemäß einem der Ansprüche 1 bis 6, dadurch gekennzeichnet, dass die Konzentration an Wirkstoff zwischen 5 und Werten höher als 10 mg/ml liegt.

8. Pharmazeutische Zusammensetzung gemäß-einem der Ansprüche 1 bis 7, dadurch gekennzeichnet, dass das ungesättigte Phospholipid ein natürliches, synthetisches oder halbsynthetisches Phospholipid ist.

9. Pharmazeutische Zusammensetzung gemäß Anspruch 8, dadurch gekennzeichnet, dass das ungesättigte Phospholipid ein natürliches Phospholipid ist.

10. Pharmazeutische Zusammensetzung gemäß Anspruch 8 oder 9, dadurch gekennzeichnet, dass das natürliche Phospholipid ein Phospholipid pflanzlichen Ursprungs, insbesondere von Sonnenblume oder von Soja, ist.

11. Pharmazeutische Zusammensetzung gemäß einem der Ansprüche 1 bis 10, dadurch gekennzeichnet, dass das ungesättigte Phospholipid aus 70 bis 100% Phosphatidylcholin besteht,

12. Pharmazeutische Zusammensetzung gemäß Anspruch 10 oder 11, dadurch gekennzeichnet, dass das natürliche Phospholipid pflanzlichen Ursprungs mehr als 70% ungesättigte Acylketten enthält.

13. Pharmazeutische Zusammensetzung gemäß einem der vorhergehenden Ansprüche, dadurch gekennzeichnet, dass das Therapeutikum der Taxoid-Klasse, das unter Docetaxel oder den Docetaxel-Derivaten ausgewählt ist, in einer Menge von 1 bis 30 Gew.-%, bezogen auf die Summe an Phospholipiden, eingeführt wird.

14. Pharmazeutische Zusammensetzung gemäß Anspruch 13, dadurch gekennzeichnet, dass das Therapeutikum in einer Menge von 3 bis 20 Gew.-%, bezogen auf die Summe an Phospholipiden, eingeführt wird.

15. Pharmazeutische Zusammensetzung gemäß Anspruch 13 oder 14, dadurch gekennzeichnet, dass das therapeutische Mittel, in einer Menge von 3,5 bis 10 Gew.-%, bezogen auf die Summe an Phospholipiden, eingeführt wird.

16. Pharmazeutische Zusammensetzung gemäß einem der vorhergehenden Ansprüche, dadurch gekennzeichnet, dass das(die) negative(n) Phospholipid(e) unter den Alkalisalzen oder quaternären Ammoniumsalzen von Phosphatidylglycerol, von Phosphatidylserin, von Phosphatidylinositol von Phosphatidsäure oder deren Derivaten ausgewählt ist(sind).

17. Pharmazeutische Zusammensetzung gemäß Anspruch 16, dadurch gekennzeichnet, dass das(die) negative(n) Phospholipid(e) in einer Menge von ungefähr 0,1 bis 4 Gew.-%, bezogen auf die Gesamtmenge des(der) ungesättigten Phospholipids(Phospholipide), eingeführt wird(werden).

18. Pharmazeutische Zusammensetzung gemäß Anspruch 16 oder 17, dadurch gekennzeichnet, dass das(die) negative(n) Phospholipid(e) in einer Menge von ungefähr 0,4 bis 0,8 Gew.-%, bezogen auf die Gesamtmenge des(der) ungesättigten Phospholipids(Phospholipide), eingeführt wird(werden).

19. Pharmazeutische Zusammensetzung gemäß einem der Ansprüche 16 bis 18, dadurch gekennzeichnet, dass das(die) negative(n) Phospholipid(e) in einer Menge von ungefähr 0,5 Gew.-%, bezogen auf die Gesamtmenge des(der) ungesättigten Phospholipids(Phospholipide), eingeführt wird(werden).

20. Pharmazeutische Zusammensetzung gemäß einem der vorhergehenden Ansprüche, dadurch gekennzeichnet, dass es sich um eine homogene Dispersion handelt.

21. Pharmazeutische Zusammensetzung gemäß einem der vorhergehenden Ansprüche, dadurch gekennzeichnet, dass sie flüssig, gefroren oder lyophilisiert sein kann.

22. Pharmazeutische Zusammensetzung gemäß einem der vorhergehenden Ansprüche, dadurch gekennzeichnet, dass sie einen Kryoprotektor und/oder ein Mittel, das dazu bestimmt ist, die Isotonie der End-Injektionslösung einzustellen, enthält.

23. Pharmazeutische Zusammensetzung gemäß Anspruch 22, dadurch gekennzeichnet, dass das Mittel unter den Zuckern, den Polymeren oder den Aminosäuren ausgewählt ist.

24. Zusammensetzung gemäß einem der vorhergehenden Ansprüche, dadurch gekennzeichnet, dass sie Teilchen enthält, deren mittlerer Durchmesser kleiner als 200 nm ist.

25. Zusammensetzung gemäß Anspruch 24, dadurch gekennzeichnet, dass sie Teilchen enthält, deren mittlerer Durchmesser kleiner als 100 nm ist.

26. Verfahren zur Herstellung einer pharmazeutischen Zusammensetzung gemäß einem der vorhergehenden Ansprüche, dadurch gekennzeichnet, dass man eine homogene Dispersion bildet durch Auflösen eines oder mehrerer ungesättigter Phospholipide, einer geringen Menge eines oder mehrerer negativer Phospholipide und eines Wirkstoffs der Taxoid-Klasse, der unter Docetaxel oder den Docetaxel-Derivaten ausgewählt ist, in Alkohol, Verdampfen des gesamten oder eines Teils des Alkohols bis zum Erhalt eines Gels oder einer viskosen Flüssigkeit, die durch Hinzufügen von Wasser unter Rühren wieder aufgenommen wird, danach Homogenisieren, man danach die erhaltene Dispersion gegebenenfalls gefriert oder lyophilisiert.

27. Verfahren gemäß Anspruch 26, dadurch gekennzeichnet, dass der Alkohol Ethanol ist.

28. Verfahren zur Herstellung einer homogenen Dispersion gemäß Anspruch 20, dadurch gekennzeichnet, dass man die nach Wasser-Zugabe erhaltene Dispersion der Phospholipide und des Wirkstoffs der Taxoid-Klasse, der unter Docetaxel oder den Docetaxelderivaten ausgewählt ist, einer Homogenisierung unterzieht.

29. Verfahren gemäß Anspruch 28, dadurch gekennzeichnet, dass die Homogenisierung in mehreren Wiederholungsschritten durchgeführt wird.

30. Verfahren gemäß einem der Ansprüche 26 bis 29, dadurch gekennzeichnet, dass die erhaltene homogene Dispersion einer Sterilfiltration unterzogen wird.

31. Verwendung einer gefrorenen oder lyophilisierten Lösung gemäß Anspruch 21 zur Herstellung von stabilen, gebrauchsfertigen Injektionslösungen.

## Claims

1. Pharmaceutical composition which is stable and highly concentrated with respect to an active principle of the taxoid class chosen from docetaxel and docetaxel derivatives, characterized in that it comprises a therapeutic agent of the taxoid class chosen from docetaxel and docetaxel derivatives, one or more unsaturated phospholipids and a small amount of one or more negative phospholipids.

2. Pharmaceutical composition according to claim 1, characterized in that it comprises 3 to 15 mg/ml of the therapeutic agent of the taxoid class chosen from docetaxel and docetaxel derivatives.

3. Pharmaceutical composition according to either of claims 1 and 2, characterized in that the therapeutic agent of the taxoid class chosen from docetaxel and docetaxel derivatives is chosen from the products of general formula: 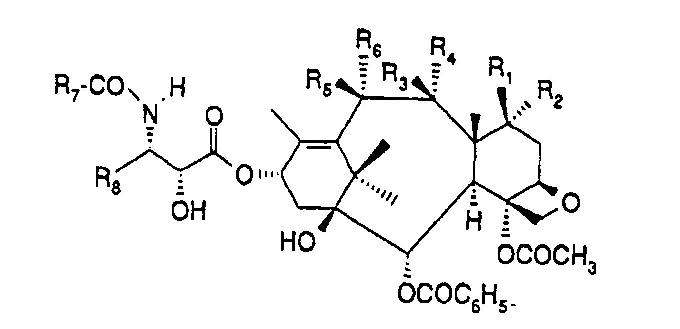 in which:
- the symbols R₁ and R₂ each represent a hydrogen atom, or alternatively one of the radicals R₁ and R₂ represents a hydrogen atom and the other represents a hydroxyl, acyloxy or acylcarbonyloxy radical, or alternatively R₂ represents a hydrogen atom and R₁ forms a bond with the carbon atom of the methyl radical at the α-position so as to form a cyclopropane ring,
- one of the symbols R₃ and R₄ represents a hydrogen atom and the other represents a hydroxyl radical, or alternatively R₃ and R₄ together form an oxo radical,
- symbols R₅ and R₆ each represent a hydrogen atom, or alternatively one of the symbols R₅ and R₆ represents a hydrogen atom and the other represents a hydroxyl, acyloxy, acylcarbonyloxy or alkoxymethylcarbonyloxy radical, or alternatively R₅ and R₆ together form an oxo radical,
- the symbol R₇ represents an alkoxy, alkenyloxy or cycloalkyloxy radical, and
- R₈ represents an unbranched or branched alkyl, unbranched or branched alkenyl or unbranched or branched alkynyl radical or a cycloalkyl radical containing 3 to 6 carbon atoms, or alternatively represents a phenyl radical optionally substituted with one or more identical or different atoms or radicals chosen from halogen atoms and alkyl, alkoxy, dialkylamino, acylamino, alkoxycarbonylamino and trifluoromethyl radicals, or a 5-membered aromatic heterocyclic radical containing one or more identical or different hetero atoms chosen from nitrogen, oxygen and sulphur atoms,
on the understanding that the alkyl radicals and the alkyl portions of the other radicals contain 1 to 8 carbon atoms in an unbranched or branched chain, and that the alkenyl or alkynyl radicals contain 2 to 8 carbon atoms.

4. Pharmaceutical composition according to claim 1, 2 or 3, characterized in that the therapeutic agent of the taxoid class chosen from docetaxel and docetaxel derivatives is chosen from the products defined in claim 2 for which, with R₂ representing a hydrogen atom, R₁ represents a hydrogen atom or a hydroxyl radical, or alternatively R₁ forms a single bond with the carbon atom of the methyl radical at the α-position, R₃ and R₄ together form an oxo radical, R₅ represents a hydrogen atom and R₆ represents a hydrogen atom or a hydroxyl, acetyloxy or methoxyacetyloxy radical, or alternatively R₅ and R₆ together form an oxo radical, R₇ represents a t-butoxy radical and R₈ represents an isobutyl, isobutenyl, butenyl, cyclohexyl, phenyl, 2-furyl, 3-furyl, 2-thienyl, 3-thienyl, 2-thiazolyl, 4-thiazolyl or 5-thiazolyl radical.

5. Pharmaceutical composition according to one of claims 1 to 4, characterized in that the therapeutic agent is docetaxel.

6. Pharmaceutical composition according to one of claims 1 to 4, characterized in that the therapeutic agent is 4α,10β-diacetoxy-2α-benzoyloxy-5β,20-epoxy-1β-hydroxy-7β,8β-methylene-9-oxo-19-nor-11-taxen-13α-yl (2R,3S)-3'-t-butoxycarbonylamino-2'-hydroxy-3'-phenylpropionate.

7. Pharmaceutical composition according to one of claims 1 to 6, characterized in that the concentration of active principle is between 5 and values above 10 mg/ml.

8. Pharmaceutical composition according to one of claims 1 to 7, characterized in that the unsaturated phospholipid is a natural, synthetic or semi-synthetic phospholipid.

9. Pharmaceutical composition according to claim 8, characterized in that the unsaturated phospholipid is a natural phospholipid.

10. Pharmaceutical composition according to claim 8 or 9, characterized in that the natural phospholipid is a phospholipid of vegetable origin, especially of sunflower or of soya bean origin.

11. Pharmaceutical composition according to one of claims 1 to 10, characterized in that the unsaturated phospholipid consists of 70 to 100 % of phosphatidylcholine.

12. Pharmaceutical composition according to claim 10 or 11, characterized in that the natural phospholipid of vegetable origin contains a level of unsaturated acyl chains of greater than 70 %.

13. Pharmaceutical composition according to one of the preceding claims, characterized in that the therapeutic agent of the taxoid class chosen from docetaxel and docetaxel derivatives is introduced in the proportion of 1 to 30 % by weight relative to the sum of the phospholipids.

14. Pharmaceutical composition according to claim 13, characterized in that the therapeutic agent is introduced in the proportion of 3 to 20 % by weight relative to the sum of the phospholipids.

15. Pharmaceutical composition according to claim 13 or 14, characterized in that the therapeutic agent is introduced in the proportion of 3.5 to 10 % by weight relative to the sum of the phospholipids.

16. Pharmaceutical composition according to one of the preceding claims, characterized in that the negative phospholipid(s) is/are chosen from the alkali metal salts or quaternary ammonium salts of phosphatidylglycerol, of phosphatidylserine, of phosphatidylinositol of phosphatidic acid or their derivatives.

17. Pharmaceutical composition according to claim 16, characterized in that the negative phospholipid(s) is/are introduced in the proportion of approximately 0.1 to 4 % by weight relative to the total amount of unsaturated phospholipid(s).

18. Pharmaceutical composition according to claim 16 or 17, characterized in that the negative phospholipid(s) is/are introduced in the proportion of approximately 0.4 to 0.8 % by weight relative to the total amount of unsaturated phospholipid(s).

19. Pharmaceutical composition according to one of claims 16 to 18, characterized in that the negative phospholipid(s) is/are introduced in the proportion of approximately 0.5 % by weight relative to the total amount of unsaturated phospholipid(s).

20. Pharmaceutical composition according to one of the preceding claims, characterized in that it is a homogeneous dispersion.

21. Pharmaceutical composition according to one of the preceding claims, characterized in that it can be liquid, frozen or lyophilized.

22. Pharmaceutical composition according to one of the preceding claims, characterized in that it contains a cryoprotective agent and/or an agent intended for adjusting the isotonicity of the final solution to be injected.

23. Pharmaceutical composition according to claim 22, characterized in that the agent is chosen from sugars, polymers and amino acids.

24. Composition according to one of the preceding claims, characterized in that it contains particles the average diameter of which is less than 200 nm.

25. Composition according to claim 24, characterized in that it contains particles the average diameter of which is less than 100 nm.

26. A process for preparing a pharmaceutical composition according to one of the preceding claims, characterized in that a homogeneous dispersion is formed by dissolution of one or more unsaturated phospholipids, a small amount of one or more negative phospholipids and an active principle of the taxoid class chosen from docetaxel and docetaxel derivatives in an alcohol, and evaporation of all or part of the alcohol until a gel or a viscous liquid is obtained, which gel or liquid is taken up by adding water with stirring and then homogenization, and the dispersion obtained is then optionally frozen or lyophilized.

27. A process according to claim 26, characterized in that the alcohol is ethanol.

28. Process for preparing a homogeneous dispersion according to claim 20, characterized in that the dispersion of phospholipids and of the active principle of the taxoid family chosen from docetaxel and docetaxel derivatives, obtained after addition of water, is subjected to a homogenization.

29. Process according to claim 28, characterized in that the homogenization is carried out in several repeated steps.

30. A process according to one of claims 26 to 29, characterized in that the homogeneous dispersion obtained is subjected to a sterilizing filtration.

31. Use of a frozen or lyophilized solution according to claim 21, for the preparation of ready-to-use, stabilized injectable solutions.
